# EUROPEAN PATENT SPECIFICATION

(11) **EP 4 056 218 B1**
(45) Date of publication and mention of the grant of the patent: **24.07.2024**
(21) Application number: 22159965.7
(22) Date of filing: 03.03.2022
(51) Int. Cl.: A61M 16/06

(54) **A MEDICAL MASK**
MEDIZINISCHE MASKE
MASQUE MÉDICAL

(30) Priority: 12.03.2021 JP 2021040786
(43) Date of publication of application: 14.09.2022
(73) Proprietor: Nihon Kohden Corporation, Shinjuku-ku Tokyo (JP)
(72) Inventor: Baba, Yuya, Tokorozawa-shi, Saitama (JP); Aoyagi, Takayuki, Tokorozawa-shi, Saitama (JP); Matsubara, Isao, Tokorozawa-shi, Saitama (JP)
(74) Representative: Grünecker Patent- und Rechtsanwälte PartG mbB

(56) References cited:
- US-A1- 2008 178 886
- US-A1- 2011 226 240
- US-A1- 2015 007 816
- US-A1- 2016 001 028
- US-B1- 6 530 373
- US-B2- 10 384 027
- US-B2- 10 653 854
- US-B2- 8 136 524
- US-B2- 8 146 595

## Description

### TECHNICAL FIELD

The presently disclosed subject matter relates to a mask. In particular, the presently disclosed subject matter relates to a medical mask for a mechanical ventilator, that can be worn by a subject.

### BACKGROUND ART

A mask disclosed in JP-T-2009-540870 has been, for example, known as a mask used for non-invasive artificial respiration, etc. The respiratory mask for artificial respiration disclosed in JP-T-2009-540870 is provided with a mask shell that is configured to cover a patient's face, and a sealing body that can be put on the user's face and that is formed by an elastic member having flexibility.

By the way, in order to prevent such a situation that air leaks through a gap between the sealing body and the mask shell, the sealing body and the mask shell need to be fixed to each other securely. In this regard, in the respiratory mask disclosed in JP-T-2009-540870, where the sealing body functions as a cushion member and the mask shell functions as a mask body part, a ring member is separately provided in the respiratory mask in order to fix the sealing body and the mask shell to each other. Due to the ring member that needs to be provided separately, the respiratory mask disclosed in JP-T-2009-540870 has a problem that the number of parts of the respiratory mask is increased and the structure of the mask is complicated.

US 8,136,524 B2 provides a disposable mask system. It relates to a disposable mask system for use with patients, e.g., adult patients.

US 2008/0178886 A1 provides an aerosol delivery mask.

US 6,530,373 B1 provides a respirator mask. A respirator mask seal and shell comprises a mask shell having a face opening, a slot for air passage, and at least one attachment portion spaced around the face opening.

US 2016/0001028 A1 provides a breathing assistance apparatus.

US 10,653,854 B2 provides a nasal mask interface assembly.

US 10,384,027 B2 provides a nasal mask for use in various positive airway pressure supply systems.

US 2015/0007816 A1 provides a cushion to frame assembly mechanism.

US 8,146,595 B2 provides an adjustable patient interface for a breathing assistance system.

US 2011/0226240 A1 provides a hood for non-invasive ventilation of patients.

### SUMMARY

The invention provides a medical mask for providing artificial respiration according to claim 1.

An object of the presently disclosed subject matter is to provide a mask having a novel structure in view of the foregoing problem. In particular, the object of the presently disclosed subject matter is to provide a mask whose number of parts is reduced and whose structure is simplified.

A mask according to an aspect of the presently disclosed subject matter can include a mask body part configured to cover a face of a subject; and a cushion member fixed to the mask body part and contacts the face of the subject. The mask body part has an inner side face at least a portion of which faces the face of the subject, an outer side face positioned on an opposite side to the inner side face, and a plurality of protrusion portions provided on the outer side face. The cushion member has a plurality of engagement portions each of which is engaged with a corresponding one of the plurality of protrusion portions.

According to the aforementioned configuration, each of the plurality of protrusion portions provided on the outer side face of the mask body part is engaged with one of the plurality of engagement portions, so that the cushion member is fixed to the mask body part. Thus, a ring member for fixing the cushion member and the mask body part to each other as in a background-art mask does not have to be separately provided in the mask, so that the number of parts of the mask can be reduced and the structure of the mask can be simplified.

### ADVANTAGEOUS EFFECTS OF INVENTION

According to the presently disclosed subject matter, it is possible to provide a mask whose number of parts is reduced and whose structure is simplified.

### BRIEF DESCRIPTION OF DRAWINGS

FIG. 1 is a perspective view illustrating the whole of a mask according to an embodiment of the presently disclosed subject matter (which will be hereinafter referred to as the present embodiment).
FIG. 2 is a sectional view of the mask according to the present embodiment.
FIG. 3 is a view illustrating an inner side face of a mask body part and an inner cup.
FIG. 4 is a front view illustrating an outer side face of the mask body part.
FIG. 5 is a side view illustrating the outer side face of the mask body part.
FIG. 6 is a perspective view illustrating a cushion member including a plurality of through holes.
FIG. 7A is an enlarged view illustrating two protrusion portions disposed in the vicinity of an engagement attachment portion in a state in which the cushion member has not been fixed to the mask body part yet.
FIG. 7B is an enlarged view illustrating a state in which the two protrusion portions disposed in the vicinity of the engagement attachment portion have been inserted into the through holes formed in the cushion member.
FIG. 8 is a view illustrating a mask body part according to a modification of the present embodiment.
FIG. 9 is a side view illustrating a mask according to the modification of the present embodiment.

### DESCRIPTION OF EMBODIMENT

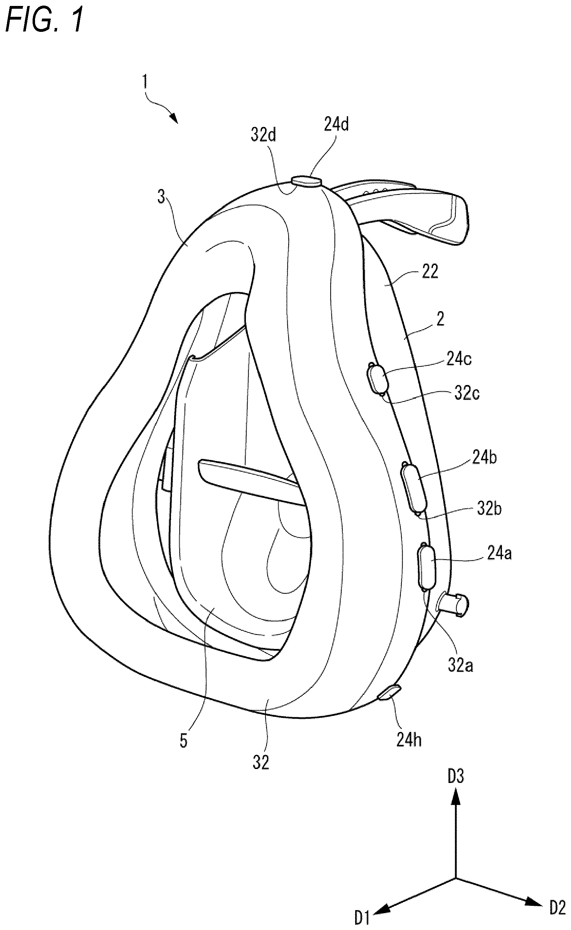

A mask 1 according to the present embodiment will be described below with reference to the drawings. In description of the present embodiment, a D1 direction, a D2 direction, and a D3 direction may be mentioned appropriately. The D1 to D3 directions are relative directions set in the mask 1 illustrated in FIG. 1. One of the D1 to D3 directions is perpendicular to the other two directions of the D1 to D3 directions.

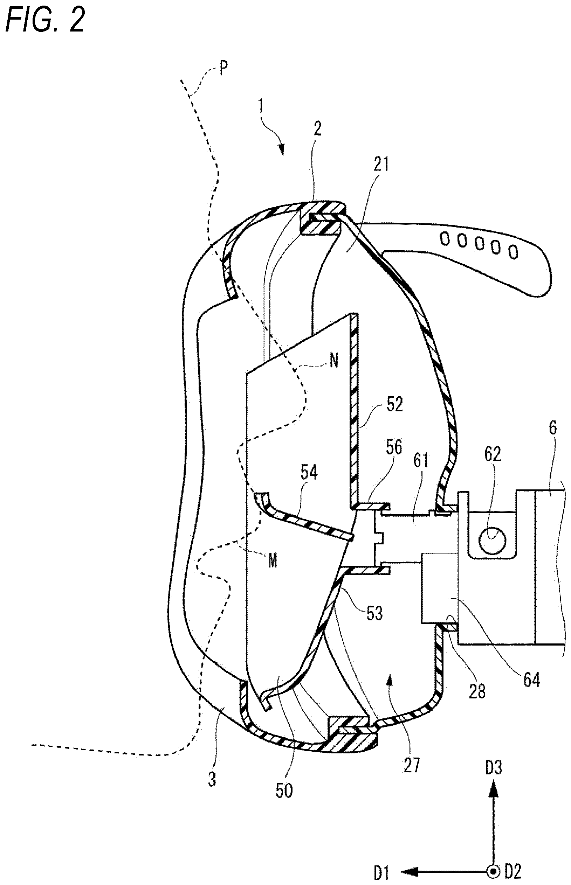

FIG. 1 is a perspective view illustrating the whole of the mask 1 according to the present embodiment. FIG. 2 is a sectional view of the mask 1. As illustrated in FIG. 1 and FIG. 2, the mask 1 is provided with a mask body part 2, a cushion member 3, and an inner cup 5 (an example of an inner part). The mask body part 2 is configured to cover a face of a subject P. The cushion member 3 is fixed to the mask body part 2. The mask 1 is, for example, a medical mask used for non-invasive artificial respiration. In particular, the mask 1 may be a medical mask applied to artificial respiration by CPAP (Continuous Positive Airway Pressure) or NPPV (Noninvasive Positive Pressure Ventilation).

The mask body part 2 is configured to cover parts of the face of the subject P (in particular, a mouth M and a nose N of the subject P). The mask body part 2 is, for example, made of a hard resin such as polycarbonate. The cushion member 3 is configured to contact the face of the subject P when the mask 1 is worn by the subject P. The cushion member 3 is, for example, made of a flexible resin material such as a silicone resin. The inner cup 5 is disposed between the mask body part 2 and the cushion member 3 in the D1 direction. In particular, the inner cup 5 is disposed in an internal space 27 defined by an inner side face 21 of the mask body part 2.

### (Configuration of Inner Cup 5)

As illustrated in FIG. 2, the inner cup 5 is configured so that exhaled gas discharged from the mouth M and the nose N of the subject P can be guided toward a joint member 6 that is connected to an exhalation sensor (not illustrated). In this respect, when the inner cup 5 is provided in the mask 1, it is possible to suitably prevent a situation that oxygen gas supplied to the subject P from a not-illustrated oxygen supply pipe through the joint member 6 and the exhaled gas discharged from the subject P are mixed with each other. Therefore, concentration of carbon dioxide contained in the exhaled gas of the subject P can be measured with high accuracy by the exhalation sensor while the oxygen gas is supplied to the subject P from the oxygen supply pipe.

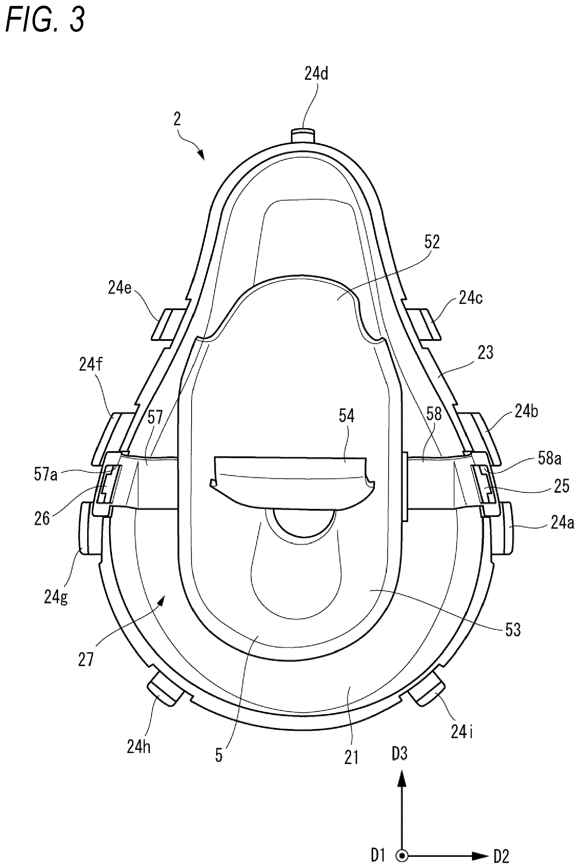

The inner cup 5 is provided with an exhalation collection cup 50 (an example of a cup), and attachment arm portions 57 and 58 (see FIG. 3). The exhalation collection cup 50 is configured to face the mouth M and the nose N of the subject P so that exhaled gas discharged from the mouth M and the nose N can be guided to the exhalation sensor. The exhalation collection cup 50 has an upper side wall portion 52, a lower side wall portion 53, an exhalation guide portion 54, and an exhalation discharge portion 56.

The upper side wall portion 52 is configured to face the nose N of the subject P so that the exhaled gas discharged from the nose N can be guided toward the exhalation discharge portion 56. The lower side wall portion 53 is configured to face the mouth M of the subject P so that the exhaled gas discharged from the mouth M can be guided toward the exhalation discharge portion 56.

The exhalation guide portion 54 is configured to extend from the exhalation discharge portion 56 toward the face of the subject P in the D1 direction so that the exhaled gas from the nose N and the exhaled gas from the mouth M can be efficiently guided to the exhalation discharge portion 56. The exhalation discharge portion 56 is connected to the upper side wall portion 52 and the lower side wall portion 53. The exhalation discharge portion 56 communicates with an exhaled gas introduction pipe 61 of the joint member 6.

The joint member 6 that has been inserted into an opening portion 28 of the mask body part 2 is fixed to the mask body part 2. The oxygen gas supplied from the oxygen supply pipe is delivered to the internal space 27 of the mask body part 2 through an oxygen gas discharge portion 64 of the joint member 6. The exhalation sensor configured to detect concentration of carbon dioxide of the exhalated gas is disposed to face an exhaled gas outlet 62 of the joint member 6. The exhalation sensor is configured to detect the concentration of the carbon dioxide of the exhaled gas discharged from the exhaled gas outlet 62.

As illustrated in FIG. 3, the attachment arm portions 57 and 58 are configured to connect the exhalation collection cup 50 to the mask body part 2. The attachment arm portions 57 and 58 face each other across the exhalation collection cup 50 in the D2 direction. The attachment arm portion 57 (an example of a first attachment arm portion) is fixed to the exhalation collection cup 50, and extends to a negative side in the D2 direction from one end side of the exhalation collection cup 50. The attachment arm portion 57 has an engagement hole portion 57a that is engaged with an engagement attachment portion 26 (an example of a first engagement attachment portion) which will be described later. The engagement hole portion 57a is formed as a through hole. The attachment arm portion 58 (an example of a second attachment arm portion) is fixed to the exhalation collection cup 50, and extends to a positive side in the D2 direction from the other end side of the exhalation collection cup 50. The attachment arm portion 58 has an engagement hole portion 58a that is engaged with an engagement attachment portion 25 (an example of a second engagement attachment portion) which will be described later. The engagement hole portion 58a is formed as a through hole.

### (Configuration of Mask Body Part 2)

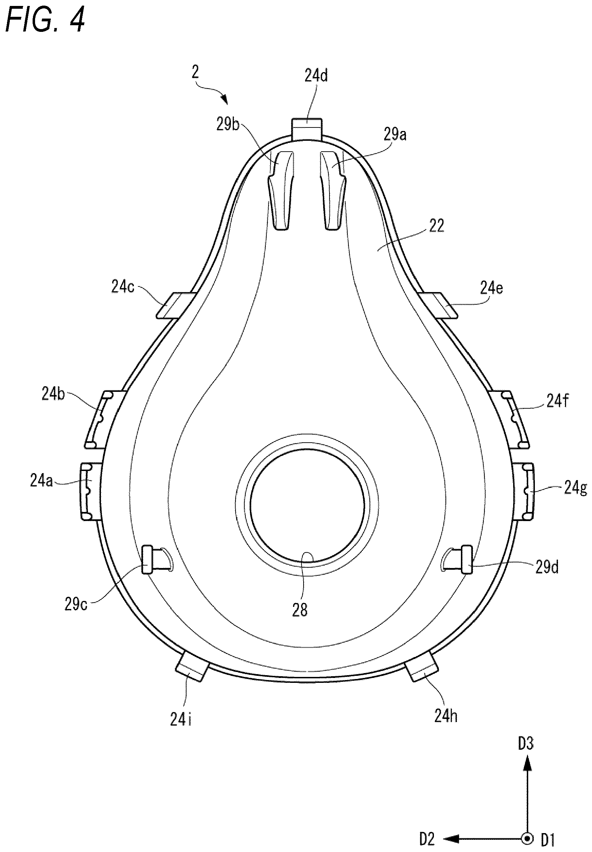

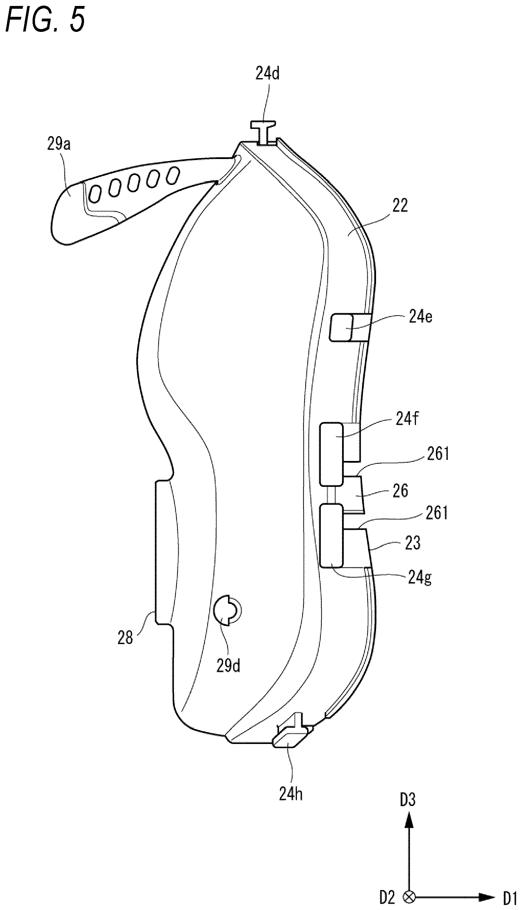

Next, the configuration of the mask body part 2 will be specifically described with reference to FIGS. 3 to 5. FIG. 3 is a view illustrating the inner side face 21 of the mask body part 2 and the inner cup 5. FIG. 4 is a front view illustrating an outer side face 22 of the mask body part 2. FIG. 5 is a side view illustrating the outer side face 22 of the mask body part 2. As illustrated in FIGS. 3 to 5, the mask body part 2 has the inner side face 21, the outer side face 22, an edge portion 23, a plurality of protrusion portions 24a to 24i, and the engagement attachment portions 25 and 26. Incidentally, in the following description, the protrusion portions 24a to 24i may be generically referred to as protrusion portions 24 simply.

The inner side face 21 defines the internal space 27 in which the inner cup 5 is received. In a state in which the mask 1 has been put on the face of the subject, the inner side face 21 partially faces the face of the subject. The outer side face 22 is positioned on an opposite side to the inner side face 21. In the state in which the mask 1 has been put on the face of the subject, the outer side face 22 fronts on the outside. The outer side face 22 partially contacts the cushion member 3 (see FIG. 1). As illustrated in FIG. 4, belt attachment portions 29a to 29d to which a not-illustrated belt is attached are provided on the outer side face 22. The belt is used to fix the mask 1 to the face of the subject.

The edge portion 23 is positioned at a boundary between the inner side face 21 and the outer side face 22 to be connected to the inner side face 21 and the outer side face 22. The plurality of protrusion portions 24a to 24i are provided on the outer side face 22. In the present embodiment, the nine protrusion portions 24a to 24i are provided on the outer side face 22 so as to extend along the edge portion 23, and intermittently surround the edge portion 23. The number of the protrusion portions 24 provided on the outer side face 22 is not particularly limited. The protrusion portion 24a faces the protrusion portion 24g across the internal space 27. The protrusion portion 24b faces the protrusion portion 24f across the internal space 27. The protrusion portion 24c is opposite to the protrusion portion 24e across the internal space 27.

The protrusion portions 24a to 24i and the belt attachment portions 29a to 29d are integrally formed with the outer side face 22 of the mask body part 2 by resin molding.

The engagement attachment portion 26 is configured to be engaged with the engagement hole portion 57a of the attachment arm portion 57. As illustrated in FIG. 5, the engagement attachment portion 26 is formed in the edge portion 23. In particular, the engagement attachment portion 26 is compartmented and configured by two grooves 261 formed in the edge portion 23. The engagement attachment portion 26 is located between the protrusion portion 24f and the protrusion portion 24g that are adjacent to each other in the D3 direction.

A direction in which the engagement attachment portion 26 extends is different from a direction in which each of the protrusion portions 24f and 24g extends. Specifically, the direction in which the engagement attachment portion 26 extends is substantially orthogonal to the direction in which the protrusion portion 24f, 24g extends. In this respect, while the engagement attachment portion 26 extends in the D1 direction, the protrusion portion 24f, 24g extends in the D2 direction.

In a circumferential direction of the edge portion 23, an interval between the protrusion portion 24f and the protrusion portion 24g is smaller than an interval between the protrusion portion 24f and the protrusion portion 24e adjacent to the protrusion portion 24f, and smaller than an interval between the protrusion portion 24g and the protrusion portion 24h adjacent to the protrusion portion 24g.

In addition, a lengthwise dimension of each of the protrusion portions 24f and 24g is larger than a lengthwise dimension of each of the protrusion portions 24e and 24h. Here, in the present embodiment, the lengthwise dimension of the protrusion portion 24 corresponds to a dimension of the protrusion portion 24 in the circumferential direction of the edge portion 23. Moreover, a widthwise dimension of the protrusion portion 24 corresponds to a dimension of the protrusion portion 24 in a direction perpendicular to the circumferential direction of the edge portion 23. A heightwise dimension of the protrusion portion 24 corresponds to a dimension of the protrusion portion 24 in a normal line direction to the outer side face 22 in the vicinity of the protrusion portion 24.

The engagement attachment portion 25 is configured to be engaged with the engagement hole portion 58a of the attachment arm portion 58. The engagement attachment portion 25 is formed in the edge portion 23. In particular, the engagement attachment portion 25 is compartmented and configured by two grooves formed in the edge portion 23. The engagement attachment portion 25 is located between the protrusion portion 24a and the protrusion portion 24b, that are adjacent to each other, in the D3 direction.

The direction in which the engagement attachment portion 25 extends is different from the direction in which each of the protrusion portions 24a and 24b extends. Specifically, the direction in which the engagement attachment portion 25 extends is substantially orthogonal to the direction in which the protrusion portion 24a, 24b extends. While the engagement attachment portion 25 extends in the D1 direction, the protrusion portion 24a, 24b extends in the D2 direction.

In the circumferential direction of the edge portion 23, an interval between the protrusion portion 24a and the protrusion portion 24b is smaller than an interval between the protrusion portion 24a and the protrusion portion 24i adjacent to the protrusion portion 24a, and smaller than an interval between the protrusion portion 24b and the protrusion portion 24c adjacent to the protrusion portion 24b. In addition, a lengthwise dimension of each of the protrusion portions 24a and 24b is larger than a lengthwise dimension of each of the protrusion portions 24c and 24i.

The inner cup 5 is fixed to the mask body part 2 in a state in which the engagement hole portion 57a of the attachment arm portion 57 is brought into engagement with the engagement attachment portion 26, and the engagement hole portion 58a of the attachment arm portion 58 is brought into engagement with the engagement attachment portion 25. Moreover, since the cushion member 3 is fixed to the mask body part 2 as illustrated in FIG. 1, movement of the inner cup 5 in the D1 direction is completely regulated.

### (Configuration of Cushion Member 3)

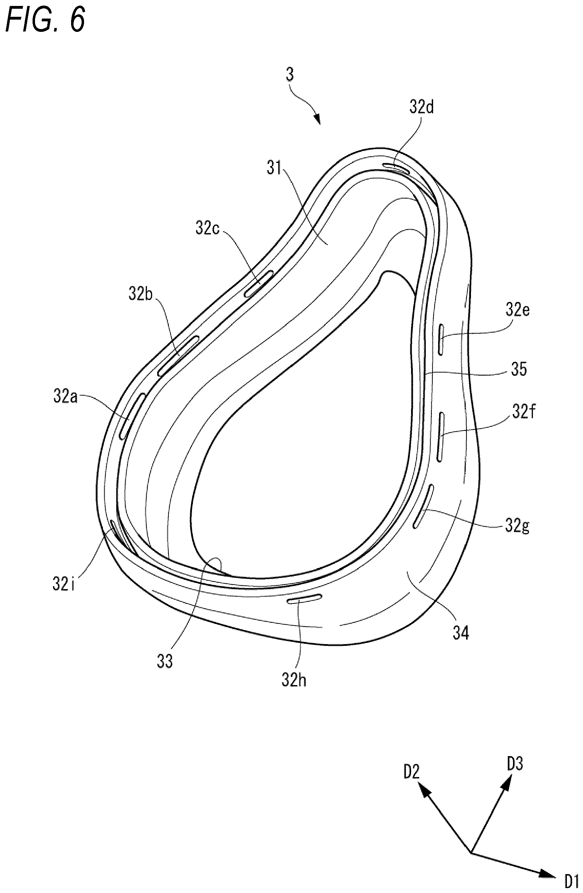

Next, the configuration of the cushion member 3 will be described below with reference to FIG. 6. The cushion member 3 has an outer side face 34 fronting on the outside, an inner side face 31 located on an opposite side to the outer side face 34, and a plurality of elongated through holes 32a to 32i (an example of engagement portions). Incidentally, in the following description, the through holes 32a to 32i may be generically referred to as through holes 32 simply.

When the mask 1 is worn by the subject, the outer side face 34 partially contacts the face of the subject. An opening portion 33 is formed in a portion of the outer side face 34 facing the face of the subject. The parts (the mouth and the nose) of the face of the subject face the inner cup 5 through the opening portion 33. The through holes 32a to 32i penetrate the cushion member 3 so as to extend from the outer side face 34 to the inner side face 31. The nine through holes 32a to 32i are provided in the outer side face 34 so as to extend along an edge portion 35 of the cushion member 3, and intermittently surround the edge portion 35. A longitudinal direction of each of the elongated through holes 32 is consistent with a circumferential direction of the edge portion 35. The number of the through holes 32 corresponds to the number of the protrusion portions 24.

When the cushion member 3 is fixed to the mask body part 2, each of the protrusion portions 24a to 24i is engaged with a corresponding one of the through holes 32a to 32i. For example, the protrusion portion 24a is engaged with the through hole 32a and the protrusion portion 24i is engaged with the through hole 32i.

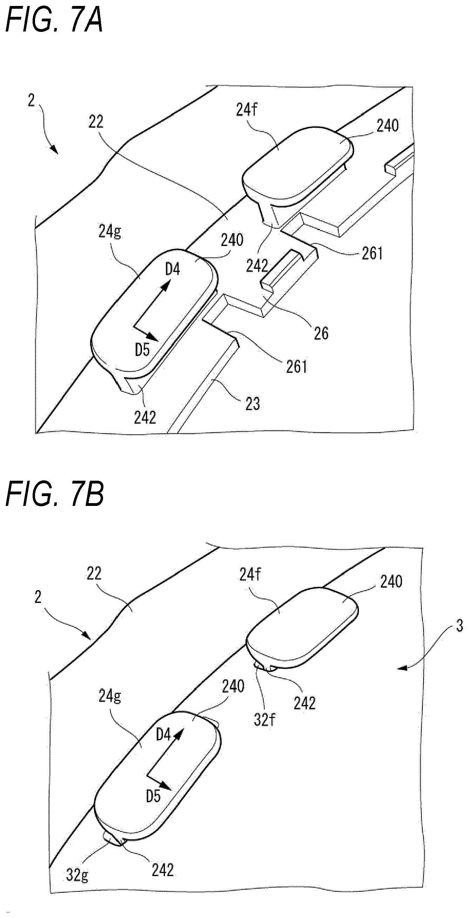

Next, the protrusion portions 24 and the through holes 32 will be described in detail with reference to FIG. 7A and FIG. 7B. FIG. 7A is an enlarged view illustrating the two protrusion portions 24f and 24g disposed in the vicinity of the engagement attachment portion 26 in a state in which the cushion member 3 has not been fixed to the mask body part 2 yet. FIG. 7B is an enlarged view illustrating a state in which the two protrusion portions 24f and 24g disposed in the vicinity of the engagement attachment portion 26 have been inserted into the through holes 32f and 32g formed in the cushion member 3.

As illustrated in FIG. 7A, each of the protrusion portions 24f and 24g has a standing wall portion 242 protruding from the outer side face 22 of the mask body part 2, and a head portion 240 connected to the standing wall portion 242. Incidentally, each of the nine protrusion portions 24a to 24i has the standing wall portion 242 and the head portion 240. One end of the standing wall portion 242 is connected to the outer side face 22 and the other end of the standing wall portion 242 is connected to the head portion 240. Each of the protrusion portions 24 is formed into a T shape when viewed from a D4 direction (corresponding to the circumferential direction of the edge portion 23) illustrated in FIG. 7A.

The head portion 240 is substantially formed into a flat face when viewed from a normal line direction of the outer side face 22 in the vicinity of the protrusion portion 24. A dimension (widthwise dimension) of the head portion 240 in a D5 direction is larger than a widthwise dimension of the standing wall portion 242. On the other hand, a dimension (lengthwise dimension) of the head portion 240 in the D4 direction is approximately equal to a lengthwise dimension of the standing wall portion 242.

When the cushion member 3 is fixed to the mask body part 2, the protrusion portion 24f is engaged with the through hole 32f of the cushion member 3 and the protrusion portion 24g is engaged with the through hole 32g of the cushion member 3, as illustrated in FIG. 7B. In this state, the cushion member 3 is held between the head portions 240 of the plurality of protrusion portions 24 and the outer side face 22 when the standing wall portions 242 of the plurality of protrusions 24 have been inserted into the plurality of through holes 32 respectively and correspondingly. Thus, the cushion member 3 is fixed to the mask body part 2 by the plurality of head portions 240 and the plurality of standing wall portions 242.

A widthwise dimension of each of the through holes 32 is smaller than the widthwise dimension of each of the head portions 240, but slightly larger than the widthwise dimension of each of the standing wall portions 242. On the other hand, a lengthwise dimension of the through hole 32 is larger than each of the lengthwise dimensions of the head portion 240 and the standing wall portion 242. According to this configuration, the widthwise dimension of the head portion 240 is larger than the widthwise dimension of the through hole 32. Accordingly, the cushion member 3 can be securely held between the head portion 240 and the outer side face 22. Moreover, since the widthwise dimension of the through hole 32 is slightly larger than the widthwise dimension of the standing wall portion 242, the standing wall portion 242 can be easily inserted into the through hole 32 so that a workload for attaching the cushion member 3 to the mask body part 2 can be reduced.

### (Effect and Function of Mask 1 According to Present Embodiment)

According to the present embodiment, the plurality of protrusion portions 24a to 24i provided on the outer side face 22 of the mask body part 2 are respectively and correspondingly engaged with the plurality of through holes 32a to 32i provided in the cushion member 3, thereby fixing the cushion member 3 to the mask body part 2. Thus, a ring member for fixing the cushion member 3 to the mask body part 2 as in the background-art mask does not need to be separately provided in the mask 1. Consequently, the number of parts of the mask 1 can be reduced and the structure of the mask 1 provided with the inner cup 5 can be simplified.

As another method for fixing the mask body part 2 and the cushion member 3 to each other, a method for fixing the mask body part 2 and the cushion member 3 to each other by an adhesive agent or a method for integrally forming the mask body part 2 and the cushion member 3 by two-color molding can be considered. On the other hand, the fixation method between the mask body part 2 and the cushion member 3 according to the present embodiment can reduce manufacturing cost of the mask 1 provided with the inner cup 5 as compared with these methods.

In addition, the plurality of protrusion portions 24 are provided on the outer side face 22 so as to extend along the edge portion 23 of the mask body part 2. Further, the cushion member 3 is partially held between the plurality of head portions 240 and the outer side face 22 in the state in which the standing wall portions 242 of the plurality of protrusion portions 24 have been inserted into the plurality of through holes 32 respectively and correspondingly. Thus, the cushion member 3 and the mask body part 2 can be fixed to each other securely by the plurality of protrusion portions 24.

Moreover, in the present embodiment, the two engagement attachment portions 25 and 26 are provided not in the outer side face 22 of the mask body part 2 but in the edge portion 23. Therefore, the direction in which the engagement attachment portion 25 extends is substantially orthogonal to the direction in which each of the protrusion portions 24a and 24b extends, and the direction in which the engagement attachment portion 26 extends is substantially orthogonal to the direction in which each of the protrusion portions 24f and 24f extends. As a result, a situation in which air leaks out through a gap between the cushion member 3 and the outer side face 22 of the mask body part 2 can be securely prevented in and around an area of the mask body part 2 where the engagement attachment portions 25 and 26 are provided. Thus, high adhesiveness between the cushion member 3 and the mask body part 2 can be ensured over the entire circumference of the edge portion 23 of the mask body part 2.

Further, the interval between the protrusion portion 24f and the protrusion portion 24g is smaller than the interval between the protrusion portion 24f and the protrusion portion 24e, and smaller than the interval between the protrusion portion 24g and the protrusion portion 24h. The lengthwise dimension of each of the protrusion portions 24f and 24g is larger than the lengthwise dimension of each of the protrusion portions 24e and 24h. In a similar manner or the same manner, the interval between the protrusion portion 24a and the protrusion portion 24b is smaller than the interval between the protrusion portion 24a and the protrusion portion 24i adjacent to the protrusion portion 24a, and smaller than the interval between the protrusion portion 24b and the protrusion portion 24c adjacent to the protrusion portion 24b. Further, the lengthwise dimension of each of the protrusion portions 24a and 24b is larger than the lengthwise dimension of each of the protrusion portions 24c and 24i.

According to the aforementioned configuration, the fixation between the cushion member 3 and the mask body part 2 can be strengthened in and around the area of the mask body part 2 where the two mounting portions 25 and 26 are provided. As a result, the fixation between the inner cup 5 and the mask body part 2 can be strengthened.

### (Mask 1a According to Modification of Present Embodiment)

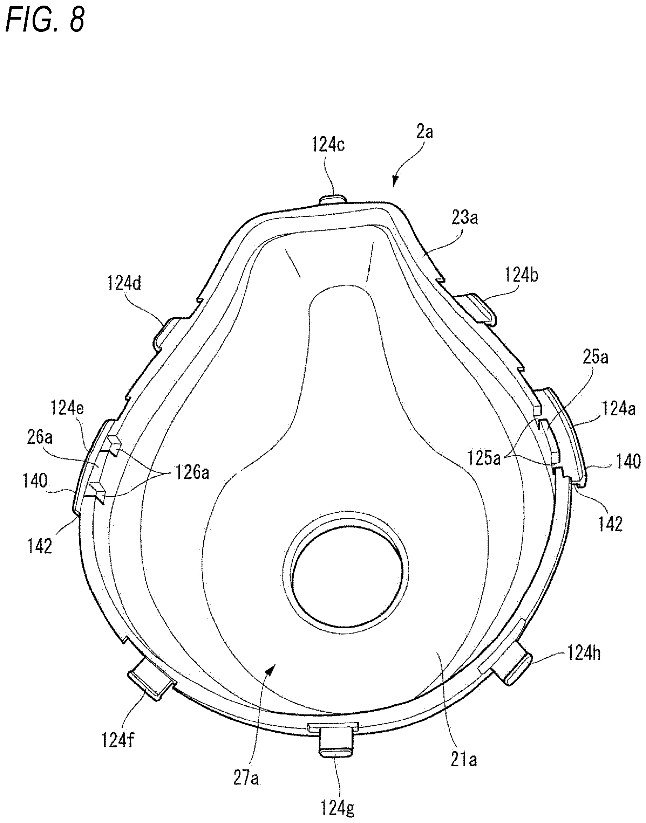

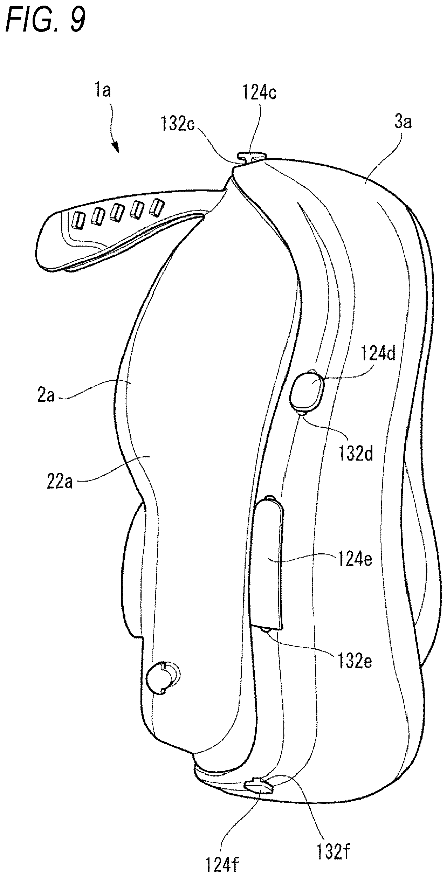

Next, a mask la according to a modification of the present embodiment will be described below with reference to FIG. 8 and FIG. 9. FIG. 8 is a view illustrating a mask body part 2a according to the modification of the present embodiment. FIG. 9 is a side view illustrating the mask la according to the modification of the present embodiment. The mask la according to the modification differs from the mask 1 according to the present embodiment in configurations of protrusion portions of the mask body part 2a. The configurations of the protrusion portions, which are the difference between the present embodiment and the modification, will be mainly described as follows.

As illustrated in FIG. 8 and FIG. 9, the mask body part 2a has an inner side face 21a, an outer side face 22a located on an opposite side to the inner side face 21a, an edge portion 23a located at a boundary between the inner side face 21a and the outer side face 22a, and protrusion portions 124a to 124h. The eight protrusion portions 124a to 124h are provided on the outer side face 22a so as to extend along the edge portion 23a, and intermittently surround the edge portion 23a. The protrusion portion 124a faces the protrusion portion 124e across an internal space 27a defined by the inner side face 21a. Each of the protrusion portions 124a to 124h has a standing wall portion 142 protruding from the outer side face 22a, and a head portion 140 connected to the standing wall portion 142.

Two engagement attachment portions 25a and 26a are formed in the edge portion 23a. The engagement attachment portion 25a is compartmented and configured by two grooves 125a. The engagement attachment portion 26a is compartmented and configured by two grooves 126a. The engagement attachment portion 25a is engaged with an attachment arm portion 57 of an inner cup 5 illustrated in FIG. 3, and the engagement attachment portion 26a is engaged with an attachment arm portion 58.

The protrusion portion 124a is provided on the outer side face 22a so as to face the engagement attachment portion 25a. A dimension (lengthwise dimension) of the protrusion portion 124a in a circumferential direction of the edge portion 23a is larger than each of lengthwise dimensions of the two protrusion portions 124b and 124h adjacent to the protrusion portion 124a. The protrusion portion 124e is provided on the outer side face 22a so as to face the engagement attachment portion 26a. A lengthwise dimension of the protrusion portion 124e is larger than each of length dimensions of the two protrusion portions 124d and 124f adjacent to the protrusion portion 124e. In particular, the lengthwise dimension of the protrusion portion 124a, 124e may be twice or more as large as the lengthwise dimension of any of the protrusion portions 124a to 124h other than the protrusion portions 124a and 124e.

Thus, the mask body part 2a according to the modification differs from the mask body part 2 according to the present embodiment in shapes of the protrusion portions formed in the vicinities of the engagement attachment portions. In particular, in the mask body part 2, two protrusion portions are provided on the outer side face in each of the vicinities of the engagement attachment portions. On the other hand, in the mask body part 2a, one protrusion portion is provided on the outer side face so as to face each of the engagement attachment portions.

As illustrated in FIG. 9, the mask la is constituted by the mask body part 2a, a cushion member 3a, and a not-illustrated inner cup. Each of the protrusion portions 124a to 124h is engaged with a corresponding one of through holes formed in the cushion member 3a, to thereby fix the cushion member 3a to the mask body part 2a. In this respect, the protrusion portions 124c to 124f are respectively and correspondingly engaged with through holes 132c to 132f in a state in which the cushion member 3a has been fixed to the mask body part 2a in the side view of the mask la illustrated in FIG. 9.

According to the present modification, the fixation between the cushion member 3a and the mask body part 2a can be strengthened in and around the area of the mask body part 2a where the engagement attachment portion 25a is provided and in and around the area of the mask body part 2a where the engagement attachment portion 26a is provided. As a result, the fixation between the inner cup and the mask body part 2a can be strengthened.

It is understood by those skilled in the art that the present embodiment is exemplified and various changes can be made on the embodiment within the scope of the invention described in Claims. The technical scope of the present invention should be defined based on the scope of the invention described in Claims and the scopes of equivalents thereto.

In the present embodiment, the through holes 32 engaged with the protrusion portions 24 of the mask body part 2 are formed along the edge portion of the cushion member 3. However, a plurality of bottomed grooves may be formed along the edge portion of the cushion member 3 in place of the through holes. Even in such a case, the cushion member 3 and the mask body part 2 can be fixed to each other when the plurality of bottomed grooves of the cushion member 3 are engaged with the plurality of protrusion portions 24 respectively and correspondingly.

Moreover, in the present embodiment, the engagement hole portions 57a and 58a provided in the attachment arm portions 57 and 58 are formed as through holes, but may be formed as bottomed grooves in place of the through holes.

Further, each of the engagement attachment portions 25 and 26 formed in the edge portion 23 is formed by the two grooves so as to extend in the D1 direction. However, the present embodiment is not limited thereto. For example, the engagement attachment portion 25, 26 may be formed as a protrusion portion protruding in the D2 direction from the inner side face 21 of the mask body part 2. Also in this case, the inner cup 5 can be fixed to the mask body part 2 when the engagement hole portions 57a and 58a formed as the through holes or the bottomed grooves are engaged with the engagement attachment portions 25 and 26 formed as the protrusion portions in a similar manner or the same manner.

Moreover, in the present embodiment, the widthwise dimension of the head portion 240 is larger than the widthwise dimension of the standing wall portion 242, but the lengthwise dimension of the head portion 240 is approximately equal to the lengthwise dimension of the standing wall portion 242, as illustrated in FIG. 7A. However, the present embodiment is not limited thereto. For example, the lengthwise dimension of the head portion 240 may be larger than the lengthwise dimension of the standing wall portion 242, but the widthwise dimension of the head portion 240 may be approximately equal to the widthwise dimension of the vertical wall portion 242. Alternatively, the lengthwise dimension of the head portion 240 may be larger than the lengthwise dimension of the standing wall portion 242, and the widthwise dimension of the head portion 240 may be larger than the widthwise dimension of the standing wall portion 242.

In addition, the inner cup 5 is received in the internal space 27 of the mask body part 2 in the present embodiment. However, the mask 1 may not be provided with the inner cup 5. Moreover, another inner member in place of the inner cup 5 may be received in the internal space 27.

## Claims

1. A medical mask (1) for providing artificial respiration, comprising:
a mask body part (2) configured to cover a face of a subject; and
a cushion member (3) fixed to the mask body part and contacts the face of the subject, wherein
the mask body part has
an inner side face (21) at least a portion of which faces the face of the subject,
an outer side face (22) positioned on an opposite side to the inner side face, and
a plurality of protrusion portions (24) provided on the outer side face; and
the cushion member (3) has a plurality of engagement portions (32) each of which is engaged with a corresponding one of the plurality of protrusion portions (24);
**characterized in that**
the mask further comprises an inner part disposed between the mask body part (2) and the cushion member (3), wherein
the inner part has
a cup (5) that faces at least one of a nose (N) and a mouth (M) of the subject (P), and
an attachment arm portion (57, 58) configured to connect the cup (5) to the mask body part (2); and
the mask body part (2) further has an engagement attachment portion (25, 26) engaged with the attachment arm portion (57, 58).

2. The mask according to Claim 1, wherein
the mask body part (2) further has an edge portion (23) located between the inner side face (21) and the outer side face (22), and connected to the inner side face and the outer side face;
the plurality of protrusion portions (24) are provided on the outer side face (22) so as to extend along the edge portion (23) of the mask body part (2); and
the plurality of engagement portions (32) are provided along the edge portion (35) of the cushion member (3).

3. The mask according to Claim 1 or 2, wherein
each of the protrusion portions (24) has
a standing wall portion (242) that protrudes from the outer side face (22), and
a head portion (240) connected to the standing wall portion (242);
one end of the standing wall portion (242) is connected to the outer side face (22), and the other end of the standing wall portion (242) is connected to the head portion (240); and
the cushion member (3) is held between the head portion (240) and the outer side face (22) in a state where the standing wall portions (242) of the protrusion portions (24) have been inserted into the engagement portions (32) respectively and correspondingly.

4. The mask according to Claim 3, wherein
a widthwise dimension of the head portion (240) is larger than a widthwise dimension of the standing wall portion (242), or a lengthwise dimension of the head portion (240) is larger than a lengthwise dimension of the standing wall portion (242).

5. The mask according to Claim 1, wherein
the mask body part (2) further has an edge portion (23) located between the inner side face (21) and the outer side face (22), and connected to the inner side face and the outer side face; and
the engagement attachment portion (25, 26) is provided in the edge portion of the mask body part.

6. The mask according to Claim 5, wherein
the engagement attachment portion (25, 26) is formed by two grooves formed in the edge portion; and
a direction in which the engagement attachment portion (25, 26) extends is different from a direction in which each of the protrusion portions (24) extends.

7. The mask according to Claim 6, wherein
the direction in which the engagement attachment portion (25, 26) extends is substantially orthogonal to the direction in which each of the protrusion portions (24) extends.

8. The mask according to any one of Claims 1 to 7, wherein
some of the protrusion portions (24) face each other across an internal space (27) of the mask body part (2) defined by the inner side face (21).

9. The mask according to any one of Claims 1 to 7, wherein
the engagement attachment portion (25, 26) is located between, of the protrusion portions (24), a first protrusion portion and a second protrusion portion that are adjacent to each other; and
an interval between the first protrusion portion and the second protrusion portion is smaller than an interval between the first protrusion portion and another protrusion portion adjacent to the first protrusion portion.

10. The mask according to Claim 9, wherein
each of lengthwise dimensions of the first protrusion portion and the second protrusion portion is larger than a lengthwise dimension of the other protrusion portion.

11. The mask according to any one of Claims 1 to 7, wherein
the attachment arm portion has
a first attachment arm portion (57), and
a second attachment arm portion (58) that faces the first attachment arm portion;
the engagement attachment portion has
a first engagement attachment portion (26) engaged with the first attachment arm portion (57), and
a second engagement attachment portion (25) engaged with the second attachment arm portion (58);
the first engagement attachment portion (26) is located between, of the protrusion portions (24), a first protrusion portion and a second protrusion portion that are adjacent to each other;
the second engagement attachment portion (25) is located between, of the protrusion portions, a third protrusion portion and a fourth protrusion portion that are adjacent to each other;
the first protrusion portion and the third protrusion portion face each other across an internal space (27) of the mask body part (2) defined by the inner side face (21); and
the second protrusion portion and the fourth protrusion portion face each other across the internal space (27).

12. The mask according to any one of Claims 1 to 7, wherein
the first protrusion portion of the protrusion portions (24) is provided on the outer side face so as to face the engagement attachment portion (26); and
a lengthwise dimension of the first protrusion portion is larger than a lengthwise dimension of another protrusion portion adjacent to the first protrusion portion.

13. The mask according to any one of Claims 1 to 7, wherein
the attachment arm portion has
a first attachment arm portion (57), and
a second attachment arm portion (58) that faces the first attachment arm portion (57);
the engagement attachment portion has
a first engagement attachment portion (26) engaged with the first attachment arm portion, and
a second engagement attachment portion (27) engaged with the second attachment arm portion;
the first engagement attachment portion (26) faces the first protrusion portion of the protrusion portions (24);
the second engagement attachment portion (25) faces the second protrusion portion of the protrusion portions (24);
the first protrusion portion and the second protrusion portion face each other across an internal space (27) of the mask body part (2) defined by the inner side face (21);
a lengthwise dimension of the first protrusion portion is larger than a lengthwise dimension of another protrusion portion adjacent to the first protrusion portion; and
a lengthwise dimension of the second protrusion portion is larger than a lengthwise dimension of another protrusion portion adjacent to the second protrusion portion.

## Patentansprüche

1. Medizinische Maske (1) für künstliche Beatmung, umfassend:
einen Maskenkörper-Teil (2), der so ausgeführt ist, dass er ein Gesicht eines Patienten abdeckt; sowie
ein Polsterelement (3), das an dem Maskenkörper-Teil befestigt ist und mit dem Gesicht des Patienten in Kontakt ist,
wobei
der Maskenkörper-Teil aufweist:
eine Innenseitenfläche (21), von der wenigstens ein Abschnitt dem Gesicht des Patienten zugewandt ist, eine Außenseitenfläche (22), die an einer der Innenseitenfläche gegenüberliegenden Seite positioniert ist, sowie
eine Vielzahl von Vorsprungsabschnitten (24), die an der Außenseitenfläche vorhanden sind; und
das Polsterelement (3) eine Vielzahl von Eingriffsabschnitten (32) aufweist, von denen jeder mit einem entsprechenden der Vielzahl von Vorsprungsabschnitten (24) in Eingriff ist;
**dadurch gekennzeichnet, dass**
die Maske des Weiteren einen inneren Teil umfasst, der zwischen dem Maskenkörper-Teil (2) und dem Polsterelement (3) angeordnet ist, wobei
der innere Teil aufweist:
eine Schale (5), die einer Nase (N) oder/und einem Mund (M) des Patienten (P) zugewandt ist, sowie
einen Anbringungsarmabschnitt (57, 58), der so ausgeführt ist, dass er die Schale (5) mit dem Maskenkörper-Teil (2) verbindet; und
der Maskenkörper-Teil (2) des Weiteren einen Eingriffs-Anbringungsabschnitt (25, 26) aufweist, der mit dem Anbringungsarmabschnitt (57, 58) in Eingriff ist.

2. Maske nach Anspruch 1, wobei
der Maskenkörper-Teil (2) des Weiteren einen Randabschnitt (23) aufweist, der sich zwischen der Innenseitenfläche (21) und der Außenseitenfläche (22) befindet und mit der Innenseitenfläche sowie der Außenseitenfläche verbunden ist;
die Vielzahl von Vorsprungsabschnitten (24) an der Außenseitenfläche (22) so vorhanden sind, dass sie sich entlang des Randabschnitts (23) des Maskenkörper-Teils (2) erstrecken; und
die Vielzahl von Eingriffsabschnitten (32) entlang des Randabschnitts (35) des Polsterelementes (3) vorhanden ist.

3. Maske nach Anspruch 1 oder 2, wobei
jeder der vorstehenden Abschnitte (24) aufweist:
einen stehenden Wandabschnitt (242), der von der Außenseitenfläche (22) vorsteht, sowie
einen Kopfabschnitt (240), der mit dem stehenden Wandabschnitt (242) verbunden ist;
ein Ende des stehenden Wandabschnitts (242) mit der Außenseitenfläche (22) verbunden ist und das andere Ende des stehenden Wandabschnitts (242) mit dem Kopfabschnitt (240) verbunden ist; und
das Polsterelement (3) zwischen dem Kopfabschnitt (240) und der Außenseitenfläche (22) in einem Zustand gehalten wird, in dem die stehenden Wandabschnitte (242) der Vorsprungsabschnitte (24) jeweils entsprechend in die Eingriffsabschnitte (32) eingeführt worden sind.

4. Maske nach Anspruch 3, wobei
eine Breitenabmessung des Kopfabschnitts (240) größer ist als eine Breitenabmessung des stehenden Wandabschnitts (242), oder eine Längsabmessung des Kopfabschnitts (240) größer ist als eine Längsabmessung des stehenden Wandabschnitts (242).

5. Maske nach Anspruch 1, wobei
der Maskenkörper-Teil (2) des Weiteren einen Randabschnitt (23) aufweist, der sich zwischen der Innenseitenfläche (21) und der Außenseitenfläche (22) befindet und mit der Innenseitenfläche sowie der Außenseitenfläche verbunden ist; und
der Eingriffs-Anbringungsabschnitt (25, 26) in dem Randabschnitt des Maskenkörper-Teils vorhanden ist.

6. Maske nach Anspruch 5, wobei
der Eingriffs-Anbringungsabschnitt (25, 26) durch zwei in dem Randabschnitt ausgebildete Nuten gebildet wird; und
eine Richtung, in der sich der Eingriffs-Anbringungsabschnitt (25, 26) erstreckt, sich von einer Richtung unterscheidet, in der sich jeder der Vorsprungsabschnitte (24) erstreckt.

7. Maske nach Anspruch 6, wobei
die Richtung, in der sich der Eingriffs-Anbringungsabschnitt (25, 26) erstreckt, im Wesentlichen orthogonal zu der Richtung ist, in der sich jeder der Vorsprungsabschnitte (24) erstreckt.

8. Maske nach einem der Ansprüche 1 bis 7, wobei
einige der Vorsprungsabschnitte (24) einander über einen Innenraum (27) des Maskenkörper-Teils (2) zugewandt sind, der durch die Innenseitenfläche (21) gebildet wird.

9. Maske nach einem der Ansprüche 1 bis 7, wobei
der Eingriffs-Anbringungsabschnitt (25, 26) sich zwischen einem ersten Vorsprungsabschnitt und einem zweiten Vorsprungsabschnitt der Vorsprungsabschnitte (24) befindet, die benachbart zueinander sind; und
ein Abstand zwischen dem ersten Vorsprungsabschnitt und dem zweiten Vorsprungsabschnitt kleiner ist als ein Abstand zwischen dem ersten Vorsprungsabschnitt und einem zu dem ersten Vorsprungsabschnitt benachbarten anderen Vorsprungsabschnitt.

10. Maske nach Anspruch 9, wobei
jede der Längsabmessungen des ersten Vorsprungsabschnitts und des zweiten Vorsprungsabschnitts größer ist als eine Längsabmessung des anderen Vorsprungsabschnitts.

11. Maske nach einem der Ansprüche 1 bis 7, wobei
der Anbringungsarmabschnitt aufweist:
einen ersten Anbringungsarmabschnitt (57), sowie
einen zweiten Anbringungsarmabschnitt (58), der dem ersten Anbringungsarmabschnitt zugewandt ist;
wobei der Eingriffs-Anbringungsabschnitt aufweist:
einen ersten Eingriffs-Anbringungsabschnitt (26), der mit dem ersten Anbringungsarmabschnitt (57) in Eingriff ist, sowie
einen zweiten Eingriffs-Anbringungsabschnitt (25), der mit dem zweiten Anbringungsarmabschnitt (58) in Eingriff ist,
wobei der erste Eingriffs-Anbringungsabschnitt (25, 26) sich zwischen einem ersten Vorsprungsabschnitt und einem zweiten Vorsprungsabschnitt der Vorsprungsabschnitte (24) befindet, die benachbart zueinander sind;
der zweite Eingriffs-Anbringungsabschnitt (25, 25) sich zwischen einem dritten Vorsprungsabschnitt und einem vierten Vorsprungsabschnitt der Vorsprungsabschnitte befindet, die benachbart zueinander sind;
der erste Vorsprungsabschnitt und der dritte Vorsprungsabschnitt einander über einen Innenraum (27) des Maskenkörper-Teils (2) zugewandt sind, der durch die Innenseitenfläche (21) gebildet wird; und
der zweite Vorsprungsabschnitt und der vierte Vorsprungsabschnitt einander über den Innenraum (27) zugewandt sind.

12. Maske nach einem der Ansprüche 1 bis 7, wobei
der erste Vorsprungsabschnitt der Vorsprungsabschnitte (24) an der Außenseitenfläche so vorhanden ist, dass er dem Eingriffs-Anbringungsabschnitt (26) zugewandt ist; und
eine Längsabmessung des ersten Vorsprungsabschnitts größer ist als eine Längsabmessung eines anderen Vorsprungsabschnitts, der benachbart zu dem ersten Vorsprungsabschnitt ist.

13. Maske nach einem der Ansprüche 1 bis 7, wobei
der Anbringungsarmabschnitt aufweist:
einen ersten Anbringungsarmabschnitt (57), sowie
einen zweiten Anbringungsarmabschnitt (58), der dem ersten Anbringungsarmabschnitt (57) zugewandt ist;
wobei der Eingriffs-Anbringungsabschnitt aufweist:
einen ersten Eingriffs-Anbringungsabschnitt (26), der mit dem ersten Anbringungsarmabschnitt in Eingriff ist, sowie
einen zweiten Eingriffs-Anbringungsabschnitt (27), der mit dem zweiten Anbringungsarmabschnitt in Eingriff ist,
wobei der erste Eingriffs-Anbringungsabschnitt (26) dem ersten Vorsprungsabschnitt der Vorsprungsabschnitte (24) zugewandt ist;
der zweite Eingriffs-Anbringungsabschnitt (25) dem zweiten Vorsprungsabschnitt der Vorsprungsabschnitte (24) zugewandt ist;
der erste Vorsprungsabschnitt und der zweite Vorsprungsabschnitt einander über einen Innenraum (27) des Maskenkörper-Teils (2) zugewandt sind, der durch die Innenseitenfläche (21) gebildet wird;
eine Längsabmessung des ersten Vorsprungsabschnitts größer ist als eine Längsabmessung eines anderen Vorsprungsabschnitts, der benachbart zu dem ersten Vorsprungsabschnitt ist; und
eine Längsabmessung des zweiten Vorsprungsabschnitts größer ist als eine Längsabmessung eines anderen Vorsprungsabschnitts, der benachbart zu dem zweiten Vorsprungsabschnitt ist.

## Revendications

1. Masque médical (1) destiné à fournir une respiration artificielle, comprenant :
une partie corps de masque (2) configurée pour recouvrir un visage d'un sujet ; et
un élément coussin (3) fixé à la partie corps de masque et contactant le visage du sujet,
la partie corps de masque ayant
une face côté interne (21) dont au moins une portion fait face au visage du sujet,
une face côté externe (22) positionnée sur un côté opposé à la face côté interne, et
une pluralité de portions faisant saillie (24) prévues sur la face côté externe ; et
l'élément coussin (3) ayant une pluralité de portions d'engagement (32) dont chacune est engagée avec l'une correspondante de la pluralité de portions faisant saillie (24) ;
**caractérisé en ce que**
le masque comprend en outre une partie interne disposée entre la partie corps de masque (2) et l'élément coussin (3),
la partie interne ayant
une coupe (5) qui fait face au moins à un nez (N) et une bouche (M) du sujet (P), et
une portion bras de fixation (57, 58) configurée pour raccorder la coupe (5) à la partie corps de masque (2) ; et
la partie corps de masque (2) ayant en outre une portion de fixation par engagement (25, 26) engagée avec la portion bras de fixation (57, 58).

2. Masque selon la revendication 1,
la partie corps de masque (2) ayant en outre une portion bord (23) localisée entre la face côté interne (21) et la face côté externe (22), et raccordée à la face côté interne et à la face côté externe ;
la pluralité de portions faisant saillie (24) étant disposées sur la face côté externe (22) afin de s'étendre le long de la portion bord (23) de la partie corps de masque (2) ; et
la pluralité de portions d'engagement (32) étant disposées le long de la portion bord (35) de l'élément coussin (3).

3. Masque selon la revendication 1 ou 2,
chacune des portions faisant saillie (24) ayant
une portion paroi stationnaire (242) qui fait saillie depuis la face côté externe (22), et
une portion tête (240) raccordée à la portion paroi stationnaire (242) ;
une extrémité de la portion paroi stationnaire (242) étant raccordée à la face côté externe (22), et l'autre extrémité de la portion paroi stationnaire (242) étant raccordée à la portion tête (240) ; et
l'élément coussin (3) étant maintenu entre la portion tête (240) et la face côté externe (22) sous un état où les portions parois stationnaires (242) des portions faisant saillie (24) ont été insérées respectivement et de manière correspondante dans les portions d'engagement (32).

4. Masque selon la revendication 3,
une dimension dans le sens de la largeur de la portion tête (240) est plus grande qu'une dimension dans le sens de la largeur de la portion paroi stationnaire (242), ou une dimension dans le sens de la longueur de la portion tête (240) est plus grande qu'une dimension dans le sens de la longueur de la portion paroi stationnaire (242).

5. Masque selon la revendication 1,
la partie corps de masque (2) ayant en outre une portion bord (23) localisée entre la face côté interne (21) et la face côté externe (22), et raccordée à la face côté interne et à la face côté externe ; et
la portion de fixation par engagement (25, 26) étant disposée dans la portion bord de la partie corps de masque.

6. Masque selon la revendication 5,
la portion de fixation par engagement (25, 26) étant formée de deux rainures formées dans la portion bord ; et
un sens dans lequel la portion de fixation par engagement (25, 26) s'étend étant différent d'un sens dans lequel chacune des portions faisant saillie (24) s'étend.

7. Masque selon la revendication 6,
le sens dans lequel la portion de fixation par engagement (25, 26) s'étend est sensiblement orthogonal au sens dans lequel chacune des portions faisant saillie (24) s'étend.

8. Masque selon l'une quelconque des revendications 1 à 7,
certaines des portions faisant saillie (24) faisant face les une aux autres à travers un espace interne (27) de la partie corps de masque (2) définie par la face côté interne (21).

9. Masque selon l'une quelconque des revendications 1 à 7,
la portion de fixation par engagement (25, 26) étant localisée entre, parmi les portions faisant saillie (24), une première portion faisant saillie et une seconde portion faisant saillie qui sont adjacentes l'une par rapport à l'autre ; et
un intervalle entre la première portion faisant saillie et la seconde portion faisant saillie est plus petit qu'un intervalle entre la première portion faisant saillie et une autre portion faisant saillie adjacente à la première portion faisant saillie.

10. Masque selon la revendication 9,
chacune des dimensions dans le sens de la longueur de la première portion faisant saillie et de la seconde portion faisant saillie étant plus grande qu'une dimension dans le sens de la longueur de l'autre portion faisant saillie.

11. Masque selon l'une quelconque des revendications 1 à 7,
la portion bras de fixation ayant
une première portion bras de fixation (57), et
une seconde portion bras de fixation (58) qui fait face à la première portion bras de fixation ;
la portion de fixation par engagement ayant
une première portion de fixation par engagement (26) engagée avec la première portion bras de fixation (57), et
une seconde portion de fixation par engagement (25) engagée avec la seconde portion bras de fixation (58) ;
la première portion de fixation par engagement (26) étant localisée entre, parmi les portions faisant saillie (24), une première portion faisant saillie et une seconde portion faisant saillie qui sont adjacentes l'une par rapport à l'autre ;
la seconde portion de fixation par engagement (25) étant localisée entre, parmi les portions faisant saillie, une troisième portion faisant saillie et une quatrième portion faisant saillie qui sont adjacentes l'une par rapport à l'autre ;
la première portion faisant saillie et la troisième portion faisant saillie se faisant face l'une l'autre à travers un espace interne (27) de la partie corps de masque (2) définie par la face côté interne (21) ; et
la seconde portion faisant saillie et la quatrième portion faisant saillie se faisant face l'une l'autre à travers l'espace interne (27).

12. Masque selon l'une quelconque des revendications 1 à 7,
la première portion faisant saillie des portions faisant saillie (24) étant disposée sur la face côté externe afin de faire face à la portion de fixation par engagement (26) ; et
une dimension dans le sens de la longueur de la première portion faisant saillie étant plus grande qu'une dimension dans le sens de la longueur d'une autre portion faisant saillie adjacente à la première portion faisant saillie.

13. Masque selon l'une quelconque des revendications 1 à 7,
la portion bras de fixation ayant
une première portion bras de fixation (57), et
une seconde portion bras de fixation (58) qui fait face à la première portion bras de fixation (57) ;
la portion de fixation par engagement ayant
une première portion de fixation par engagement (26) engagée avec la première portion bras de fixation, et
une seconde portion de fixation par engagement (27) engagée avec la seconde portion bras de fixation ;
la première portion de fixation par engagement (26) faisant face à la première portion faisant saillie des portions faisant saillie (24) ;
la seconde portion de fixation par engagement (25) faisant face à la seconde portion faisant saillie des portions faisant saillie (24) ;
la première portion faisant saillie et la seconde portion faisant saillie se faisant face l'une l'autre à travers un espace interne (27) de la partie corps de masque (2) définie par la face côté interne (21) ;
une dimension dans le sens de la longueur de la première portion faisant saillie étant plus grande qu'une dimension dans le sens de la longueur d'une autre portion faisant saillie adjacente à la première portion faisant saillie ; et
une dimension dans le sens de la longueur de la seconde portion faisant saillie étant plus grande qu'une dimension dans le sens de la longueur d'une autre portion faisant saillie adjacente à la seconde portion faisant saillie.
